# FASCICULE DE BREVET EUROPEEN

(11) **EP 2 099 739 B1**
(45) Date de publication et mention de la délivrance du brevet: **24.11.2010**
(21) Numéro de dépôt: 07866549.4
(22) Date de dépôt: 30.11.2007
(51) Int. Cl.: C07C 213/06, C07C 219/08

(54) **PROCEDE DE SYNTHESE D'ESTERS (METH)ACRYLIQUES CATALYSE PAR UN TITANATE DE POLYOL.**
VERFAHREN ZUR SYNTHESE VON DURCH EIN POLYOLTITANAT KATALYSIERTEN (METH)ACRYLISCHEN ESTERN
METHOD FOR THE SYNTHESIS OF (METH)ACRYLIC ESTERS CATALYSED BY A POLYOL TITANATE.

(30) Priorité: 05.12.2006 FR 0655306
(43) Date de publication de la demande: 16.09.2009
(73) Titulaire: ARKEMA FRANCE, 92700 Colombes (FR)
(72) Inventeur: PAUL, Jean-Michel, 57070 Metz (FR)
(74) Mandataire: Bonnel, Claudine
(86) Numéro de dépôt international: PCT/FR2007/052429
(87) Numéro de publication internationale: WO 2008/068444

(56) Documents cités:
- EP-A2- 0 298 867
- SCHURRENBERGER P ET AL: "Herstellung von Methylestern durch Umesterung funktionalisierter Substrate mit Titalsäureestern als Katalysatoren" HELVETICA CHIMICA ACTA, vol. 65, no. 4, 1982, pages 1197-1201, XP002439899 ISSN: 0018-019X cité dans la demande
- LEWIS N ET AL: "A highly efficient preparation of methacrylate esters using novel solid phase titanium-based transesterification catalysts" SYNLETT 1999 GERMANY, no. SPEC. ISS., 1999, pages 957-959, XP002439898 ISSN: 0936-5214 cité dans la demande
- DELEUZE H ET AL: "Polymer-supported titanates as catalysts for transesterification reactions" POLYMER, ELSEVIER SCIENCE PUBLISHERS B.V, GB, vol. 39, no. 24, novembre 1998 (1998-11), pages 6109-6114, XP004132841 ISSN: 0032-3861 cité dans la demande

## Description

La présente invention concerne un procédé de synthèse d'esters (méth)acryliques par transestérification en utilisant un catalyseur à base de titanate de polyol.

Un procédé couramment utilisé pour produire des esters (méth)acryliques est la transestérification. II est connu, par exemple d'après les documents GB 960 005, EP 298 867, EP 619 309 ou EP 960 877, de préparer les esters (méth)acryliques de formule (I) : 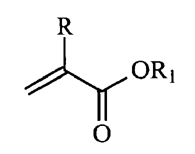 dans laquelle R est un atome d'hydrogène ou un groupement méthyle, et R₁ pouvant être un radical alkyle linéaire ou ramifié, ou un radical aliphatique cyclique, aryle, alkyle-aryle ou aryle-alkyle, pouvant contenir des hétéroatomes, selon un procédé de transestérification par réaction d'un (méth)acrylate d'alkyle de formule (II) : 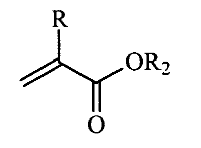 dans laquelle R a la signification précitée et R₂ pouvant être un groupement alkyle linéaire ou ramifié ayant de 1 à 4 atomes de carbone, avec un alcool de formule (III) :

R₁-OH

dans laquelle R₁ a la signification précitée.

Au cours de la synthèse est généré de l'alcool léger R₂-OH, qui est éliminé sous forme d'un azéotrope avec le (méth)acrylate d'alkyle léger (II).

La synthèse des esters (méth)acryliques par transestérification s'effectue généralement en présence d'un catalyseur. Le choix du catalyseur dépend de différents critères, notamment de la nature du (méth)acrylate d'alkyle (II) mis en oeuvre, mais aussi de la nature de l'alcool (III) utilisé pour préparer l'ester

(méth)acrylique. En plus des critères d'efficacité et de sélectivité, d'autres facteurs peuvent intervenir, tels que la disponibilité commerciale, le prix ou la toxicité du catalyseur.

Comme exemples de catalyseurs décrits dans la littérature pour catalyser la préparation d'esters (méth)acryliques par transestérification, on peut citer notamment :
- Des catalyseurs acides, tels que l'acide méthane sulfonique, ou l'acide para toluène sulfonique : ces catalyseurs ont l'inconvénient d'être peu sélectifs et corrosifs.
- Des catalyseurs basiques, tels que des sels alcalins ou alcalino-terreux : ces catalyseurs sont actifs mais s'avèrent peu sélectifs.
- Les chélates de titane, zirconium, fer, zinc, ou calcium avec des composés 1,3-dicarbonyie comme les acétyiacétonates de Ti, Zr, Fr, Zn ou Ca : ces composés sont actifs et sélectifs, mais génèrent de l'acétylacétone et sont sensibles à la présence de diols 1,2 ou 1,3. De plus, ils perdent rapidement leur activité et ne sont pas de ce fait recyclables.
- Les dérivés de l'étain, tels que les dialkyloxydes d'étain, les dialkyldialcoxydes d'étain et les dialkyldiesters d'étain, en particulier le di-n-butyloxyde d'étain (DBTO et ses homologues), sont généralement actifs et sélectifs. Cependant, avec ces catalyseurs se pose le problème de leur élimination dans les résidus de distillation du fait de la toxicité de l'étain.
- Les alcoolates de titane, comme les titanates de tétraalkyle (éthyle, n-propyle, isopropyle, n-butyle,...), ou encore le titanate de diméthylaminoéthyle ou le phénolate de titane : ce sont des catalyseurs généralement actifs et sélectifs, mais sensibles à l'eau.
- Des enzymes dont l'utilisation est décrite dans le brevet JP 04 079889.

Les alcoolates de titane se sont révélés des catalyseurs actifs et sélectifs et leur utilisation est préconisée en particulier dans des procédés de synthèse de (méth)acrylates de dialkylaminoalkyle.

On peut citer le brevet européen EP 298 867 qui décrit notamment la préparation d'acrylate de N,N-diméthylaminoéthyle (ADAME) en présence de titanate de tétraéthyle selon un procédé de transestérification par réaction de l'acrylate d'éthyle sur le diméthylaminoéthanol.

Dans le brevet EP 960 877, les (méth)acrylates de dialkylaminoalkyle sont obtenus en présence d'un catalyseur de transestérification choisi parmi les titanates de tétrabutyle, de tétraéthyle et de tétra(2-éthylhexyle) à partir de (méth)acrylate de méthyle ou d'éthyle avec un aminoalcool. Pour obtenir un produit de grande pureté, il est cependant nécessaire de procéder d'abord à un équeutage, c'est-à-dire une élimination du catalyseur et des produits lourds, suivi d'un étêtage et d'une rectification finale du mélange brut réactionnel.

Lorsqu'il s'agit de produire un (méth)acrylate de dialkylaminoalkyle, tel que l'ADAME, par transestérification à partir de (méth)acrylate de méthyle, il est difficilement envisageable d'utiliser un alcoolate de titane tel que le titanate de tétraéthyle, en raison de l'apparition progressive d'un précipité blanc, totalement insoluble dans le milieu réactionnel, qui se révèle être du titanate de tétraméthyle. En effet, en présence du méthanol généré lors de la transestérification, il y a échange de ligands avec le titanate utilisé et formation de titanates de méthyle dont le titanate de tétraméthyle, insoluble, est le terme ultime. Outre le phénomène de désactivation du catalyseur, et par conséquent le ralentissement de la cinétique réactionnelle, il se produit aussi un encrassement du réacteur et de ses annexes, ce qui n'est pas acceptable à l'échelle industrielle.

Dans l'article POLYMER, vol 39, n°24, (1998), pages 6109-6114, Deleuze H. et Al décrivent la transestérification de l'acrylate de méthyle par le 2-éthyl hexanol en présence d'un catalyseur supporté qui se présente sous forme de billes de polymère sur lesquelles sont greffées des fonctions titanates, l'intérêt étant de pouvoir séparer facilement le catalyseur du milieu réactionnel par filtration.

L'article de Lewis N. et Al dans SYNLETT, N°Spec.ISS, 1999, pages 957-959, décrit la transestérification du méthacrylate de méthyle par le bromoundécanol en présence d'un catalyseur consistant en un titanate greffé sur un support polystyrène, facilement séparable du milieu réactionnel par simple filtration.

Il a maintenant été trouvé une famille de catalyseurs à base de titane et de polyol, permettant de catalyser la synthèse d'esters (méth)acryliques par transestérification, en particulier, à partir de (méth)acrylate de méthyle, sans observer de phénomène de désactivation du catalyseur et d'encrassement du réacteur. Ces catalyseurs, outre le fait d'être très actifs et sélectifs, présentent l'avantage de ne pas laisser précipiter de solide au cours de la réaction contrairement aux titanates d'alkyle précités qui génèrent du titanate de méthyle insoluble en présence de méthanol. La réaction d'échange de ligands avec l'alcool léger généré au cours de la réaction, tel que par exemple le méthanol, ou avec l'alcool mis en oeuvre pour la réaction ne conduit pas à des espèces insolubles dans le milieu réactionnel. Les problèmes liés aux impuretés générées par la transestérification du catalyseur sont ainsi évités, et la purification de l'ester (méth)acrylique, simplifiée. Par rapport aux catalyseurs classiques à base d'étain, les titanates de polyols présentent l'avantage de ne pas être toxiques, ce qui facilite la destruction des résidus ultimes de distillation par incinération.

La présente invention a donc pour objet un procédé de synthèse d'esters (méth)acryliques de formule (I) : 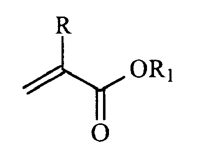 dans laquelle R est un atome d'hydrogène ou un groupement méthyle, et R₁ est un radical alkyle linéaire ou ramifié, ou un radical aliphatique cyclique, aryle, alkyle-aryle ou aryle-alkyle, comportant de 5 à 40 atomes de carbone, ou un radical alkyle linéaire ou ramifié contenant au moins un hétéroatome et de 3 à 40 atomes de carbone, par réaction d'un (méth)acrylate d'alkyle de formule (II) : 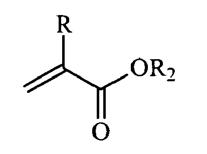 dans laquelle R a la signification précitée et R₂ est un groupement méthyle, avec un alcool de formule (III) :

R₁-OH

dans laquelle R₁ a la signification précitée,
en présence d'un catalyseur de transestérification et d'au moins un inhibiteur de polymérisation, **caractérisé en ce que** le catalyseur est un titanate de polyol de formule (IV) [(R'O)₃TiO]ₓR" dans laquelle R' est un radical alkyle linéaire ou ramifié ayant de 2 à 8 atomes de carbone pouvant contenir des hétéroatomes ou R' est le radical phényle, R" est un groupement alkylène ayant de 2 à 12 atomes de carbone, linéaire ou ramifié et pouvant comporter des atomes d'oxygène et/ou des cycles aliphatiques ou aromatiques, provenant d'un polyol R"(OH)ₓ comportant x fonctions alcool et x est un entier allant de 2 à 6.

Les titanates de polyol de formule (IV) peuvent être préparés en faisant réagir un alcoolate de titane Ti(OR')₄ avec un polyol R"(OH)ₓ, x représentant le nombre de fonctions alcool du polyol, selon la réaction (A) :

x Ti(OR')₄ + R"(OH)ₓ → [(R'O)₃TiO]ₓR" + x R'OH (A)

La réaction s'effectue de préférence en milieu solvant, l'échange de ligands se faisant en éliminant l'alcool léger R'OH généré par distillation, éventuellement sous forme d'un azéotrope avec le solvant. L'échange de ligands peut générer au cours de la réaction d'autres formes de titanates de polyol comme sous-produits.

La formation d'un titanate d'éthylène glycol, appelé Gly-Ti, à partir de titanate de tétraéthyle et d'éthylène glycol selon la réaction :

2 Ti(OC₂H₅)₄ + HO(CH₂)₂OH → (C₂H₅O)₃TiO(CH₂)₂OTi(OC₂H₅)₃ + 2 C₂H₅OH

a été décrite par Seebach dans l'article Helvetica Chimica Acta, Vol 65, Fasc.4 (1982)-Nr.110 pages 1197-1201.

En utilisant le Gly-Ti comme catalyseur, Seebach a pu préparer des esters méthyliques non acryliques à partir d'esters non acryliques et de méthanol, sans générer de titanate de tétraméthyle insoluble.

Comme solvants utilisables pour la réaction (A), on peut citer de manière non limitative le cyclohexane, le toluène, l'heptane, l'hexane.

La réaction (A) est effectuée à la température d'ébullition de l'alcool R'OH ou de l'azéotrope solvant/R'OH à la pression de service.

La réaction peut être effectuée à pression atmosphérique ou sous vide.

Après élimination complète du solvant, le composé formé est utilisable comme catalyseur.

Dans la formule (IV), le radical R' est à titre d'exemple, le radical éthyle, n-propyle, isopropyle, n-butyle, isobutyle, 2-éthylhexyle, diméthylaminoéthyle ou phényle, ainsi que leurs mélanges.

Comme titanates Ti(OR')₄ de départ, on peut utiliser à titre d'exemples les titanates de tétraalkyle tels que le titanate de tétraéthyle, le titanate de tétrapropyle, le titanate de tétraisopropyle, le titanate de tétrabutyle, le titanate de tétraisobutyle, le titanate de tétra2-éthylhexyle, ou encore le titanate de diméthylaminoéthyle ou le phénolate de titane, ainsi que leurs mélanges.

Les polyols R"(OH)ₓ utilisables pour préparer les titanates de polyol sont de fonctionnalité x allant de 2 à 6, de préférence on choisira des diols ou des triols.

A titre d'exemples de polyols, on peut citer : l'éthylène glycol, le propylène glycol, le 1,3-butanediol, le 1,4-butanediol, le 1,6-hexanediol, le néopentyl glycol, le diéthylène glycol, le triéthylène glycol, le tétraéthylène glycol, le glycérol, le dipropylèneglycol, le tripropylèneglycol, le cyclohexane diméthanol, le bisphénol-A, le triméthylolpropane, le pentaérythritol, le di-triméthylolpropane, le dipentaérythritol.

De préférence, on utilisera les diols pour lesquels le radical R" est un groupement alkylène linéaire ayant de 2 à 6 atomes de carbones pouvant comporter des atomes d'oxygène, tels que l'éthylène glycol, le diéthylène glycol, le 1,3-butanediol, le 1,4-butanediol ou le 1,6-hexanediol.

Le rapport molaire entre le titanate Ti(OR')₄ et le polyol R"(OH)ₓ selon la réaction (A) est égal au nombre de fonctions alcool du polyol. De préférence, on utilisera un rapport molaire légèrement supérieur pour un diol, de préférence compris entre 2,2 et 2 pour un diol, et légèrement inférieur pour les polyols comportant plus de deux fonctions alcool, soit par exemple un rapport molaire compris entre 2,8 et 3 pour un triol, ou compris entre 3,8 et 4 pour un polyol comportant quatre fonctions alcool.

Les titanates de polyol de formule (IV) peuvent être sous forme liquide ou solide. On préfère les titanates de polyol liquides.

Les titanates de polyol de formule (IV) sont particulièrement bien adaptés à la synthèse des composés de formule (I) dans laquelle R₁ est un radical diakylaminoalkyle provenant d'un aminoalcool de formule (R₃)(R₄)N-A-OH dans laquelle R₃ et R₄, identiques ou différents, sont un radical alkyle contenant 1 à 5 atomes de carbone, et A est un radical alkylène, linéaire ou ramifié, comportant de 1 à 5 atomes de carbone.

Le procédé selon l'invention est utilisé notamment pour préparer l'acrylate de diméthylaminoéthyle (ADAME), l'acrylate de diméthylaminopropyle, l'acrylate de diéthylaminoéthyle, l'acrylate de tertiobutylaminoéthyle, le méthacrylate de diméthylaminoéthyle (MADAME).

De préférence, le (méth)acrylate d'alkyle léger (II) est l'acrylate de méthyle.

Au cours de la synthèse est généré du méthanol, qui est éliminé sous forme d'un azéotrope avec le (méth)acrylate de méthyle (II).

Dans le procédé selon l'invention, le catalyseur est utilisé à raison de 0,001 à 0,02 mole, de préférence de 0,005 à 0,01 mole par mole d'alcool (III).

On choisit de préférence un rapport molaire (méth)acrylate de méthyle (II) sur alcool (III) compris entre 2 et 5, de préférence entre 2 et 4.

La température de réaction est généralement comprise entre 80 et 110°C et la pression est généralement maintenue entre 0,025 bar et la pression atmosphérique. De préférence, la température de réaction est comprise entre 90 et 110°C et la pression est de l'ordre de 0,5 bar à la pression atmosphérique.

Comme inhibiteur de polymérisation, on utilise la phénothiazine, l'hydroquinone, l'éther monométhylique d'hydroquinone, le diterbutyl para-crésol (BHT), la paraphénylène diamine, le TEMPO (2,2,6,6-tétraméthyl-1-piperidinyloxy), le di-tertiobutylcatéchol, ou les dérivés du TEMPO, seuls ou en mélange, à raison de 100 à 5000 ppm par rapport à la charge initiale, de préférence entre 500 et 3000 ppm.

Le procédé selon l'invention est particulièrement avantageux puisqu'il n'y a pas formation de titanate de tétraméthyle insoluble au sein du mélange réactionnel. Les rendements du procédé selon l'invention par rapport à l'alcool (III) sont élevés et le plus souvent supérieurs à 95 %. Les résidus ultimes de distillation contenant le catalyseur qui ne présente pas le caractère toxique des catalyseurs à base d'étain, peuvent être incinérés sans difficulté.

Les exemples suivants illustrent la présente invention sans toutefois en limiter la portée. Les pourcentages sont exprimés en pourcentages massiques.

### Exemple 1 : Préparation d'un catalyseur TIPOL1

Dans un réacteur agité, chauffé par circulation d'huile thermostatée dans une double enveloppe, surmonté d'une colonne à distiller à garnissage Multiknit, avec condenseur à eau glycolée en tête de colonne, tête de reflux, séparateur à vide, recettes et pièges, on charge :
- 200 g d'un mélange constitué de 80 % de titanate de tétraéthyle et 20 % de titanate de tétraisopropyle (commercialisé sous le nom VERTEC AC 560 de JOHNSON MATTHEY)
- 24,8 g d'éthylène glycol
- 300 g de cyclohexane

Le mélange réactionnel est chauffé à reflux à pression atmosphérique pendant 3 h. Les alcools légers, éthanol et isopropanol, libérés lors de l'échange de ligands sont éliminés par distillation sous forme d'azéotropes avec le cyclohexane.

Le cyclohexane résiduel est ensuite distillé. On obtient en final 195 g de produit brut qui se présente sous forme d'un liquide orange légèrement visqueux (TIPOL1).

### Exemple 2 : Synthèse d'acrylate de diméthylaminoéthyle (ADAME)

Dans le réacteur décrit à l'exemple 1, on charge :
- 595 g d'acrylate de méthyle (AM)
- 173,5 g de N,N diméthylaminoéthanol
- 1,5 g (2000 ppm par rapport à la charge) de phénothiazine
- 0,75 g (1000 ppm par rapport à la charge) de BHT (diterbutyl para crésol).

Durant toute la synthèse, on effectue un bullage d'air dans le mélange réactionnel.

L'eau présente dans la charge, provenant de l'alcool, est éliminée par distillation à pression atmosphérique sous forme d'un azéotrope AM/eau. Après la phase précédente de séchage, on introduit un complément d'acrylate de méthyle correspondant à la masse de la fraction de séchage. On ajoute ensuite 6,85 g (0,008 mole) de TIPOL1.

La réaction est effectuée à pression atmosphérique avec une température dans le milieu réactionnel comprise entre 85 et 95°C. Le méthanol formé au cours de la réaction est éliminé au fur et à mesure de sa formation sous forme d'un azéotrope AM/méthanol à une température de tête de colonne comprise entre 61,9 et 63,5°C. Le taux de conversion est suivi par analyse chromatographique de l'azéotrope. Le taux de conversion atteint 97,8 % après 270 min de mise en réaction.

Le produit brut obtenu à ce stade de la synthèse est parfaitement limpide. On n'y décèle pas la présence de solide. Le rendement et la sélectivité de l'opération sont respectivement de 96,6 et 98,7 %.

### Exemple 3 (comparatif) :

L'exemple 2 est repris en utilisant toute chose égale par ailleurs, du titanate de tétraéthyle comme catalyseur (4,1g).

Après 10 h de mise en réaction, le taux de conversion du N,N diméthylaminoéthanol est de 70 % et on observe un dépôt solide dans le réacteur qui se révèle être du titanate de méthyle insoluble.

### Exemple 4 (comparatif) :

L'exemple 2 est repris en utilisant toute chose égale par ailleurs, du titanate de tétraisopropyle comme catalyseur (4,5g).

Après 5 h de mise en réaction à une température comprise entre 84 et 88°C, le taux de conversion du N,N diméthylaminoéthanol est de 92 %. Comme à l'exemple 3, on observe un dépôt dans le produit brut réactionnel qui se révèle être du titanate de méthyle insoluble.

### exemple 5 :

Deux titanates de polyol, appelés TIPOL2 et TIPOL3 sont préparés dans les conditions de l'exemple 1 à partir de titanate d'isopropyle et respectivement d'éthylène glycol et de propylène glycol avec un rapport molaire titanate d'isopropyle/ diol = 2/1.

Ces deux titanates de polyol sont ensuite utilisés comme catalyseurs pour la synthèse de l'ADAME selon le protocole mis en oeuvre à l'exemple 2.

Les produits bruts de réaction obtenus avec ces catalyseurs sont parfaitement liquides et limpides.

Les taux de conversion et sélectivité sont identiques avec TIPOL2 ou TIPOL3 et respectivement de 98% et 96%.

## Revendications

1. Procédé de synthèse d'esters (méth)acryliques de formule (I) : 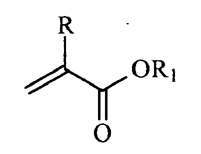 dans laquelle R est un atome d'hydrogène ou un groupement méthyle, et R₁ est un radical alkyle linéaire ou ramifié, ou un radical aliphatique cyclique, aryle, alkyle-aryle ou aryle-alkyle, comportant de 5 à 40 atomes de carbone, ou un radical alkyle linéaire ou ramifié contenant au moins un hétéroatome et de 3 à 40 atomes de carbone,
par réaction d'un (méth)acrylate d'alkyle de formule (II) : 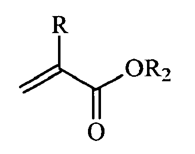 dans laquelle R a la signification précitée et R₂ est un groupement méthyle, avec un alcool de formule (III) :
R₁-OH
dans laquelle R₁ a la signification précitée,
en présence d'un catalyseur de transestérification et d'au moins un inhibiteur de polymérisation, **caractérisé en ce que** le catalyseur est un titanate de polyol de formule (IV) [(R'O)₃TiO]ₓR" dans laquelle R' est un radical alkyle linéaire ou ramifié ayant de 2 à 8 atomes de carbone pouvant contenir des hétéroatomes ou R' est le radical phényle, R" est un groupement alkylène ayant de 2 à 12 atomes de carbone, linéaire ou ramifié et pouvant comporter des atomes d'oxygène et/ou des cycles aliphatiques ou aromatiques, provenant d'un polyol R"(OH)ₓ comportant x fonctions alcool et x est un entier allant de 2 à 6.

2. Procédé selon la revendication 1, **caractérisé en ce que** le catalyseur de formule (IV) est préparé par réaction d'un alcoolate de titane Ti(OR')₄ avec un polyol R"(OH)ₓ x représentant le nombre de fonctions alcool du polyol, selon la réaction (A),
x Ti(OR')₄ + R"(OH)ₓ → [(R'O)₃TiO]ₓR" + x R'OH (A)
de préférence en milieu solvant.

3. Procédé selon la revendication 1 ou 2, **caractérisé en ce que** dans la formule (IV), le radical R" est un groupement alkylène linéaire ayant de 2 à 6 atomes de carbones pouvant comporter des atomes d'oxygène.

4. Procédé selon l'une des revendications 1 à 3, **caractérisé en ce que** dans la formule (IV), le radical R" provient d'un diol ou d'un triol.

5. Procédé selon l'une des revendications 1 à 4, **caractérisé en ce que** le radical R" provient de l'éthylène glycol, le diéthylène glycol, le 1,3-butanediol, le 1,4-butanediol ou le 1,6-hexanediol.

6. Procédé selon l'une des revendications 1 à 5, **caractérisé en ce que** dans la formule (IV), le radical R' est éthyle, n-propyle, isopropyle, n-butyle, isobutyle, 2-éthylhexyle, diméthylaminoéthyle ou phényle, ainsi que leurs mélanges.

7. Procédé selon l'une des revendications 1 à 6, **caractérisé en ce que** le catalyseur est utilisé à raison de 0,001 à 0,02 mole par mole d'alcool ( III ).

8. Procédé selon l'une des revendications 1 à 7, **caractérisé en ce que** dans la formule (I), le radical R₁ est un radical diakylaminoalkyle provenant d'un aminoalcool de formule (R₃)(R₄)N-A-OH dans laquelle R₃ et R₄, identiques ou différents, sont un radical alkyle contenant 1 à 5 atomes de carbone, et A est un radical alkylène, linéaire ou ramifié, comportant de 1 à 5 atomes de carbone.

9. Procédé selon l'une des revendications 1 à 8, pour préparer l'acrylate de diméthylaminoéthyle (ADAME), l'acrylate de diméthylaminopropyle, l'acrylate de diéthylaminoéthyle, l'acrylate de tertiobutylaminoéthyle, le méthacrylate de diméthylaminoéthyle (MADAME).

## Claims

1. A method for the synthesis of (meth)acrylic esters of formule (I): 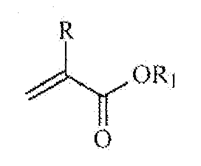 in which R is a hydrogen atom or a methyl group, and R₁ is a linear or branched alkyl radical, or a cyclic aliphatic, aryl, alkylaryl or arylalkyl radical, containing from 5 to 40 carbon atoms, or a Linear or branched alkyl radical containing at least one heteroatom and having from 3 to 40 carbon atoms,
by reacting an alkyl (meth)acrylate of formula (II) : 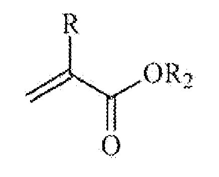 in which R has the abovementioned meaning and R₂ is a methyl group,
with an alcohol of formula (III):
R₁-OH
in which R₁ has the abovementioned meaning,
in the presence of a transesterification catalyst and of at least one polymerization inhibitor, **characterized in that** the catalyst is a polyol titanate of formula (IV) [(R'O)₃TiO]ₓR" in which R' is a linear or branched alkyl radical having from 2 to 8 carbon atoms which may contain heteroatoms or R' is a phenyl radical, R" is a linear or branched alkylene group having from 2 to 12 carbon atoms and which may comprise oxygen atoms and/or aliphatic or aromatic rings, originating from a polyol R"(OH)ₓ comprising x alcohol functions and x is an integer ranging from 2 to 6.

2. The method as claimed in claim 1, **characterized in that** the catalyst of formula (IV) is prepared by reacting a titanium alkoxide Ti(OR')₄ with a polyol R"(OH)ₓ, x representing the number of alcohol functions of the polyol, according to the reaction (A) :
x Ti(OR')₄ + R"(OH)ₓ → [(R'O)₃TiO]ₓR" + xR'OH (A)
preferably in a solvent medium.

3. The method as claimed in claim 1 or 2, **characterized in that**, in formula (IV), the R" radical is a linear alkylene group having from 2 to 6 carbon atoms which may comprise oxygen atoms.

4. The method as claimed in one of claims 1 to 3, **characterized in that**, in formula (IV), the R" radical originates from a diol or a triol.

5. The method as claimed in one of claims 1 to 4, **characterized in that** the R" radical originates from ethylene glycol, diethylene glycol, 1,3-butanediol, 1,4-butanediol or 1,6-hexanediol.

6. The method as claimed in one of claims 1 to 5, **characterized in that**, in formula (IV), the R' radical is ethyl, n-propyl, isopropyl, n-butyl, isobutyl, 2-ethylhexyl, dimethylaminoethyl or phenyl, or mixtures thereof.

7. The method as claimed in one of claims 1 to 6, **characterized in that** the catalyst is used in a proportion of from 0.001 to 0.02 mol per mole of alcohol (III).

8. The method as claimed in one of claims 1 to 7, **characterized in that**, in formula (I), the R₁ radical is to dialkylaminoalkyl radical originating from an amino alcohol of formula (R₃) (R₄)N-A-OH in which R₃ and R₄, which may be identical or different, are an alkyl radical containing 1 to 5 carbon atoms, and A is a linear or branched alkylene radical containing from 1 to 5 carbon atoms.

9. The method as claimed in one of claims 1 to 8, for preparing dimethylaminoethyl acrylate (DMAEA), dimethylaminopropyl acrylate, diethylaminoethyl acrylate, tert-butylaminoethyl acrylate or dimethylaminoethyl methacrylate (DMAEMA).

## Patentansprüche

1. Verfahren zur Synthese von (Meth)acrylsäureestern der Formel (I): 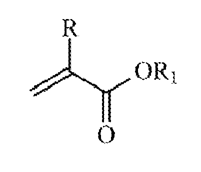 worin die Gruppe R ein Wasserstoffatom oder die Methylgruppe bedeutet und R₁ eine geradkettige oder verzweigte Alkylgruppe oder eine cyclische aliphatische Gruppe, eine Arylgruppe, eine Alkylarylgruppe oder eine Arylalkylgruppe, die 5 bis 40 Kohlenstoffatome aufweisen, oder eine geradkettige oder verzweigte Alkylgruppe bedeutet, die mindestens ein Heteroatom enthält und 3 bis 40 Kohlenstoffatome aufweist,
durch Umsetzung eines Alkyl(meth)acrylats der Formel (II): 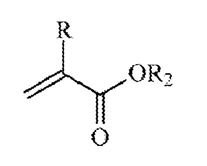 worin R die oben angegebenen Bedeutungen aufweist und R₂ die Methylgruppe bedeutet,
mit einem Alkohol der Formel (III):
R₁-OH
worin R₁ die oben angegebenen Bedeutungen aufweist,
in Gegenwart eines Umesterungskatalysators und mindestens eines Polymerisationsinhibitors, **dadurch gekennzeichnet, dass** es sich bei dem Katalysator um ein Polyoltitanat der Formel (IV) handelt: [(R'O)₃TiO]ₓR", worin R' eine geradkettige oder verzweigte Alkylgruppe mit 2 bis 8 Kohlenstoffatomen ist, die Heteroatome enthalten kann, oder R' eine Phenylgruppe bedeutet; und R" eine geradkettige oder verzweigte Alkylengruppe mit 2 bis 12 Kohlenstoffatomen ist, die Sauerstoffatome und/oder aliphatische oder aromatische Ringe enthalten kann und die von einem Polyol R"(OH)ₓ stammt, das x Alkoholfunktionen enthält, wobei x eine ganze Zahl im Bereich von 2 bis 6 bedeutet.

2. Verfahren nach Anspruch 1, **dadurch gekennzeichnet, dass** der Katalysator der Formel (IV) durch Umsetzung eines Titanalkoholats Ti(OR')₄ mit einem Polyol R"(OH)ₓ, wobei x die Anzahl der Alkoholfunktionen des Polyols angibt, gemäß der folgenden Umsetzung (A) vorzugsweise in einem Lösungsmittelmedium hergestellt wird:
x Ti(OR')₄ + R"(OH)ₓ → [(R'O)₃TiO]ₓR" + x R'OH (A)

3. Verfahren nach Anspruch 1 oder 2, **dadurch gekennzeichnet, dass** in der Formel (IV) die Gruppe R" eine lineare Alkylengruppe mit 2 bis 6 Kohlenstoffatomen bedeutet, die Sauerstoffatome enthalten kann.

4. Verfahren nach einem der Ansprüche 1 bis 3, **dadurch gekennzeichnet**, das in der Formel (IV) die Gruppe R" von einem Diol oder einem Triol stammt.

5. Verfahren nach einem der Ansprüche 1 bis 4, **dadurch gekennzeichnet, dass** die Gruppe R" von Ethylenglycol, Diethylenglycol, 1,3-Butandiol, 1,4-Butandiol oder 1,6 Hexandiol abgeleitet ist.

6. Verfahren nach einem der Ansprüche 1 bis 5, **dadurch gekennzeichnet, dass** in der Formel (IV) die Gruppe R' Ethyl, n-Propyl, Isopropyl, n-Butyl, Isobutyl, 2-Ethylhexyl, Dimethylaminoethyl oder Phenyl sowie deren Gemische bedeutet.

7. Verfahren nach einem der Ansprüche 1 bis 6, **dadurch gekennzeichnet, dass** der Katalysator in einer Menge von 0,001 bis 0,02 mol pro Mol Alkohol (III) verwendet wird.

8. Verfahren nach einem der Ansprüche 1 bis 7, **dadurch gekennzeichnet, dass** in der Formel (I) die Gruppe R₁ eine Dialkylaminoalkylgruppe ist, die von einem Aminoalkohol der Formel (R₃)(R₄)N-A-OH abgeleitet ist, worin R₃ und R₄, die gleich oder verschieden sind, eine Alkylgruppe mit 1 bis 5 Kohlenstoffatomen bedeuten und A eine geradkettige oder verzeigte Alkylengruppe mit 1 bis 5 Kohlenstoffatomen ist.

9. Verfahren nach einem der Ansprüche 1 bis 8 zur Herstellung von Dimethylaminoethylacrylat (ADAME), Dimethylaminopropylacrylat, Diethylaminoethylacrylat, tert-Butylaminoethylacrylat, Dimethylaminoethylmethacrylat (MADAME).
